# EUROPEAN PATENT SPECIFICATION

(11) **EP 1 283 042 B1**
(45) Date of publication and mention of the grant of the patent: **08.10.2008**
(21) Application number: 01932087.8
(22) Date of filing: 16.05.2001
(51) Int. Cl.: A61K 31/436, A61K 9/08, A61K 47/22, A61P 29/00, A61P 27/02, C07D 491/052, A61K 47/20

(54) **AQUEOUS LIQUID PREPARATION OF PRANOPROFEN**
WÄSSRIGE FLÜSSIGE PREPARATION VON PRANOPROFEN
PREPARATION LIQUIDE AQUEUSE DE PRANOPROFENE

(30) Priority: 17.05.2000 JP 2000145640
(43) Date of publication of application: 12.02.2003
(73) Proprietor: Senju Pharmaceutical Co., Ltd., Osaka-shi, Osaka 541-0046 (JP)
(72) Inventor: NAKAYAMA, Hisayuki, Nishinomiya-shi, Hyogo 662-0826 (JP); NEMOTO, Fukiko, Kobe-shi, Hyogo 651-2116 (JP)
(74) Representative: von Kreisler, Alek
(86) International application number: PCT/JP2001/004083
(87) International publication number: WO 2001/087303

(56) References cited:
- WO-A1-96/32941
- GB-A- 2 154 876
- JP-A- 5 186 349
- JP-A- 10 236 951
- JP-A- 10 295 777

## Description

### Technical Field

The present invention relates to an aqueous solution containing pranoprofen or a pharmacologically acceptable salt thereof, and a sulfa drug. Moreover, the present invention relates to a method for making pranoprofen or a pharmacologically acceptable salt thereof stable to light in an aqueous solution, which comprises adding a sulfa drug.

### Background Art

Pranoprofen (chemical name: α-methyl-5H-[1]benzopyrano[2,3-b]pyridine-7-acetic acid) is a nonsteroidal antiinflammatory agent useful for inflammatory diseases in outer ocular areas and anterior segments of the eye, such as conjunctivitis, keratitis, blepharitis and the like. However, pranoprofen is associated with a problem since it is unstable to light in an aqueous solution. As a method for improving the light-stability of pranoprofen in an aqueous solution, there is known only one method including concomitant use of an antioxidant, such as dibutylhydroxytoluene and the like, in an aqueous solution of pranoprofen in the presence of a nonionic surfactant (Japanese Patent No. 3021312).

### Disclosure of the Invention

It is an object of the present invention to provide a stabilized aqueous solution containing pranoprofen or a pharmacologically acceptable salt thereof. Another object of the present invention is to provide a method for making pranoprofen or a pharmacologically acceptable salt thereof stable to light in an aqueous solution containing pranoprofen or a pharmacologically acceptable salt thereof.

As a result of intensive studies conducted by the present inventors in an attempt to achieve the above-mentioned objects, it has been found that the objects can be achieved by adding a sulfa drug to an aqueous solution containing pranoprofen or a pharmacologically acceptable salt thereof, which resulted in the completion of the present invention.

According to the invention the sulfa drug is represented by a bacteriostatic pharmaceutical agent containing a sulfanilamido group.

Accordingly, the present invention relates to the following.
(1) An aqueous solution containing pranoprofen or a pharmacologically acceptable salt thereof, and a bacteriostatic pharmaceutical agent containing a sulfanilamido group
(2) The aqueous solution according to the above-mentioned (1), wherein the pranoprofen or a pharmacologically acceptable salt thereof is contained at a concentration of pranoprofen of 0.01-2.0 w/v%, and the bacteriostatic pharmaceutical agent containing a sulfanilamido group at a concentration of 2.0-4.0 w/v%.
(3) The aqueous solution according to the above-mentioned (1) wherein the bacteriostatic pharmaceutical agent containing a sulfanilamido group is at least one kind selected from the group consisting of sulfamethoxazole, sulfamonomethoxine, sulfisoxazole, sulfisomidine and pharmacologically acceptable salts thereof.
(4) The aqueous solution according to the above-mentioned (1), wherein the bacteriostatic pharmaceutical agent containing a sulfanilamido group is sulfamethoxazole or a sodium salt thereof.
(5) The aqueous solution according to the above-mentioned (1), which is an eye drop.
(6) A method for making pranoprofen or a pharmacologically acceptable salt thereof stable to light in an aqueous solution, which comprises adding a bacteriostatic pharmaceutical agent containing a sulfanilamido group to the aqueous solution containing pranoprofen or a pharmacologically acceptable salt thereof.
(7) The method according to the above-mentioned (6), wherein the bacteriostatic pharmaceutical agent containing a sulfanilamido group is added at a concentration of 2.0-4.0 w/v% to the aqueous solution containing pranoprofen or a pharmacologically acceptable salt thereof at a concentration of pranoprofen of 0.01-2.0 w/v%.
(8) The method according to the above-mentioned (6), wherein the bacteriostatic pharmaceutical agent containing a sulfanilamido group is at least one kind selected from the group consisting of sulfamethoxazole, sulfamonomethoxine, sulfisoxazole, sulfisomidine and pharmacologically acceptable salts thereof.
(9) The method according to the above-mentioned (6), wherein the bacteriostatic agent containing a sulfanilamido group the sulfa drug is sulfamethoxazole or a sodium salt thereof.

### Detailed Description of the Invention

The present invention provides a method for making pranoprofen or a pharmacologically acceptable salt thereof stable to light in an aqueous solution, which comprises adding said sulfa drug to the aqueous solution containing pranoprofen or a pharmacologically acceptable salt thereof, and an aqueous solution stabilized by such method.

In the present invention, the pharmacologically acceptable salt of pranoprofen is exemplified by, but not limited to, metal salts such as sodium salt, potassium salt, calcium salt, magnesium salt, aluminum salt and the like, and salts with an organic base such as triethylamine, diethylamine, morpholine, piperazine and the like.

In the present invention, the content of pranoprofen or a pharmacologically acceptable salt thereof as pranoprofen is generally 0.01-2.0 w/v%, preferably 0.05-1.0 w/v%.

In the present invention, the "sulfa drug" means a bacteriostatic pharmaceutical agent containing a sulfanilamide group, which is exemplified by, but not limited to, sulfamethoxazole, sulfamonomethoxine, sulfisoxazole, sulfadiazine, sulfadimethoxine, sulfamethizole, sulfisomidine, pharmacologically acceptable salts thereof and the like. Examples of the pharmacologically acceptable salt include monovalent metal salts such as sodium salt and potassium salt. Particularly preferred are sulfamethoxazole and a sodium salt thereof. The sulfa drug is used at a concentration of generally 2.0-4.0 w/v% in the present invention.

The aqueous solution of the present invention may contain, besides the above-mentioned active ingredients, antiphlogistics such as dipotassium glycyrrhizinate, allantoin, ε-aminocaproic acid and the like, vitamins such as flavin adenin dinucleotide sodium, cyanocobalamin, pyridoxine hydrochloride, tocopherol acetate and the like, amino acids such as aspartic acid, aminoethylsulfonic acid and the like, and anticholinesterase agents such as neostigmine methylsulfate and the like, as necessary.

The aqueous solution of the present invention may contain additives such as buffer, isotonicity agent, dissolution aids, preservative, viscous base, chelating agent and refrigerant, as appropriate.

Examples of the buffer include phosphate buffer, borate buffer, citrate buffer, tartrate buffer, acetate buffer, amino acid and the like.

Examples of the isotonicity agent include saccharides such as sorbitol, glucose, mannitol and the like, polyhydric alcohols such as glycerin, propylene glycol and the like, salts such as sodium chloride and the like, boric acid and the like.

Examples of the dissolution aids include nonionic surfactants such as polyoxyethylene sorbitan monooleate, polyoxyethylene hydrogenated castor oil, tyloxapol, pluronic and the like, polyhydric alcohols such as glycerin, macrogol and the like, and the like.

Examples of the preservative include quaternary ammonium salts such as benzalkonium chloride, benzethonium chloride, cetylpyridinium chloride and the like, parahydroxybenzoic acid esters such as methyl parahydroxybenzoate, ethyl parahydroxybenzoate, propyl parahydroxybenzoate, butyl parahydroxybenzoate and the like, benzyl alcohol, sorbic acid, thimerosal, chlorobutanol, sodium dehydroacetate and the like.

Examples of the viscous base include water-soluble polymers such as polyvinylpyrrolidone, polyethylene glycol, polyvinyl alcohol and the like, celluloses such as hydroxyethylcellulose, methylcellulose, hydroxypropylmethylcellulose, carboxymethylcellulose sodium and the like, and the like.

Examples of the chelating agent include sodium edetate, citric acid and the like, and examples of the refrigerant include 1-menthol, borneol, camphor, eucalyptus oil and the like.

The pH of the aqueous solution of the present invention is generally adjusted to 6.0-9.0, preferably 7.0-8.5.

The aqueous solution of the present invention may be produced according to a preparation method known per se, and can be produced according to a method described in, for example, The Japanese Pharmacopoeia thirteenth Edition, General Rules for Preparations, Liquids and Solutions, Injections and Ophthalmic Solutions.

When the aqueous solution of the present invention is used as an eye drop, it can be applied to trachoma, pink eye (acute conjunctivitis caused by bacteria or virus), hordeolum, blepharitis, conjunctivitis, keratitis, corneal ulcer, dacryocystitis and the like. The dosage thereof in the case of an eye drop of the present invention, which contains sulfamethoxazole in a proportion of 4.0 w/v% and pranoprofen in a proportion of 0.05 w/v%, may be 3-6 times of daily instillation of the eye drop (1-2 drops per instillation). The frequency of instillation can be increased or decreased appropriately depending on the degree of symptom.

### Examples

The present invention is described in more detail in the following by means of Experimental Examples and Examples.

### Experimental Example: Light-stability test of pranoprofen

### Experimental method

Aqueous solutions A and B having the formulations shown in Table 1 were prepared and filled in 15 ml transparent polyethylene terephthalate containers. Using a xenon long-life fade meter (FAL-25AX-Ec, manufactured by SUGA TEST INSTRUMENTS Co., Ltd.), the pranoprofen content after exposure to light under the following conditions was measured by HPLC method. A transparent polyethylene terephthalate container shielded from light was exposed to light in the same manner and measured for a pranoprofen content, which was used as a control.
dose: 205 W/cm²
exposure time: 2 hours

**Table 1**

| | A | B |
|---|---|---|
| pranoprofen | 0.05 g | 0.05 g |
| sulfamethoxazole | 4.0 g | - |
| sodium hydroxide | q.s. | q.s. |
| sterile purified water | ad 100 ml | ad 100 ml |
| pH | 8.3 | 8.3 |

### Test results

The pranoprofen contents after exposure to light are shown in Table 2. The residual ratio of pranoprofen in Formulation A containing sulfamethoxazole was 98.4% for the light non-shielded container and 98.8% for the light shielded container. In contrast, the residual ratio of pranoprofen in Formulation B without sulfamethoxazole was 64.0% for the light non-shielded container and 100.2% for the light shielded container. The results indicate that addition of sulfamethoxazole to an aqueous solution of pranoprofen improved stability of pranoprofen under light exposure conditions.

**Table 2**

| Formulation | Preservation conditions | Property | Residual ratio (%) |
|---|---|---|---|
| A | without light shield | clear | 98.4 |
| | light shielded | clear | 98.8 |
| B | without light shield | clouded | 64.0 |
| | light shielded | clear | 100.2 |

### Example 1

An aqueous solution for eye drop was prepared by a conventional method according to the following formulation.

| | |
|---|---|
| Pranoprofen | 0.05 g |
| sulfamethoxazole | 4.0 g |
| sodium edetate | 0.001 g |
| sodium hydroxide | q.s. |
| sterile purified water | ad 100 ml |
| pH | 8.3 |

### Example 2

An aqueous solution for eye drop was prepared by a conventional method according to the following formulation.

| | |
|---|---|
| Pranoprofen | 0.05 g |
| sulfamethoxazole | 4.0 g |
| dipotassium glycyrrhizinate | 0.1 g |
| sodium edetate | 0.001 g |
| sodium hydroxide | q.s. |
| sterile purified water | ad 100 ml |
| pH | 8.3 |

### Example 3

An aqueous solution for eye drop was prepared by a conventional method according to the following formulation.

| | |
|---|---|
| Pranoprofen | 0.05 g |
| sulfisoxazole | 4.0 g |
| sodium edetate | 0.001 g |
| sodium hydroxide | q.s. |
| sterile purified water | ad 100 ml |
| pH | 8.0 |

### Example 4

An aqueous solution for eye drop was prepared by a conventional method according to the following formulation.

| | |
|---|---|
| sodium salt of pranoprofen | 0.055 g |
| sulfamethoxazole | 4.0 g |
| sodium edetate | 0.001 g |
| sodium hydroxide | q.s. |
| sterile purified water | ad 100 ml |
| pH | 8.3 |

### Example 5

An aqueous solution for eye drop was prepared by a conventional method according to the following formulation.

| | |
|---|---|
| Pranoprofen | 0.05 g |
| sulfamethoxazole | 4.0 g |
| sodium edetate | 0.001 g |
| potassium sorbate | 0.1 g |
| sodium hydroxide | q.s. |
| sterile purified water | ad 100 ml |
| pH | 8.3 |

### Industrial Applicability

By adding a sulfa drug to an aqueous solution containing pranoprofen or a pharmacologically acceptable salt according to the present invention, the light-stability of pranoprofen or a pharmacologically acceptable salt in an aqueous solution can be improved, thereby enabling preparation of a stable aqueous solution of pranoprofen.

## Claims

1. An aqueous solution comprising pranoprofen or a pharmacologically acceptable salt thereof, and a bacteriostatic pharmaceutical agent containing a sulfanilamido group.

2. The aqueous solution according to claim 1, wherein the pranoprofen or a pharmacologically acceptable salt thereof is contained at a concentration of pranoprofen of 0.01-2.0 w/v%, and the bacteriostatic pharmaceutical agent containing a sulfanilamido group at a concentration of 2.0-4.0 w/v%.

3. The aqueous solution according to claim 1, wherein the bacteriostatic pharmaceutical agent containing a sulfanilamido group is at least one kind selected from the group consisting of sulfamethoxazole, sulfamonomethoxine, sulfisoxazole, sulfisomidine and pharmacologically acceptable salts thereof.

4. The aqueous solution according to claim 1, wherein the bacteriostatic pharmaceutical agent containing a sulfanilamido group is sulfamethoxazole or a sodium salt thereof.

5. The aqueous solution according to claim 1, which is an eye drop.

6. A method for making pranoprofen or a pharmacologically acceptable salt thereof stable to light in an aqueous solution, which comprises adding a bacteriostatic pharmaceutical agent containing a sulfanilamido group to the aqueous solution containing pranoprofen or a pharmacologically acceptable salt thereof.

7. The method according to claim 6, wherein the bacteriostatic pharmaceutical agent containing a sulfanilamido group is added at a concentration of 2.0-4.0 w/v% to the aqueous solution comprising pranoprofen or a pharmacologically acceptable salt thereof at a concentration of pranoprofen of 0.01-2.0 w/v%.

8. The method according to claim 6, wherein the bacteriostatic pharmaceutical agent containing a sulfanilamido group is at least one kind selected from the group consisting of sulfamethoxazole, sulfamonomethoxine, sulfisoxazole, sulfisomidine and pharmacologically acceptable salts thereof.

9. The method according to claim 6, wherein the bacteriostatic pharmaceutical agent containing a sulfanilamido group is sulfamethoxazole or a sodium salt thereof.

10. Use of a bacteriostatic pharmaceutical agent containing a sulfanilamido group for making pranoprofen or a pharmacologically acceptable salt thereof stable to light in an aqueous solution.

## Patentansprüche

1. Wässrige Lösung, umfassend Pranoprofen oder ein pharmakologisch annehmbares Salz davon und ein bakteriostatisches Pharmakon, das eine Sulfanilamidogruppe enthält.

2. Wässrige Lösung gemäß Anspruch 1, wobei das Pranoprofen oder sein pharmakologisch annehmbares Salz in einer Konzentration von 0,01-2,0% (w/v) Pranoprofen enthalten ist und das bakteriostatische Pharmakon, das eine Sulfanilamidogruppe enthält, in einer Konzentration von 2,0-4,0% (w/v) enthalten ist.

3. Wässrige Lösung gemäß Anspruch 1, wobei es sich bei dem bakteriostatischen Pharmakon, das eine Sulfanilamidogruppe enthält, um wenigstens eine Art handelt, die aus der Gruppe ausgewählt ist, die aus Sulfamethoxazol, Sulfamonomethoxin, Sulfisoxazol, Sulfisomidin und pharmakologisch annehmbaren Salzen davon besteht.

4. Wässrige Lösung gemäß Anspruch 1, wobei es sich bei dem bakteriostatischen Pharmakon, das eine Sulfanilamidogruppe enthält, um Sulfamethoxazol oder ein Natriumsalz davon handelt.

5. Wässrige Lösung gemäß Anspruch 1, bei der es sich um Augentropfen handelt.

6. Verfahren zur Herstellung einer lichtstabilen wässrigen Lösung von Pranoprofen oder eines pharmakologisch annehmbaren Salzes davon, umfassend das Hinzufügen eines bakteriostatischen Pharmakons, das eine Sulfanilamidogruppe enthält, zu einer wässrigen Lösung, die Pranoprofen oder ein pharmakologisch annehmbares Salz davon enthält.

7. Verfahren gemäß Anspruch 6, wobei das bakteriostatische pharmazeutische Mittel, das eine Sulfanilamidogruppe enthält, in einer Konzentration von 2,0-4,0% (w/v) zu der wässrigen Lösung gegeben wird, die Pranoprofen oder ein pharmakologisch annehmbares Salz davon in einer Konzentration von 0,01-2,0% (w/v) Pranoprofen enthält.

8. Verfahren gemäß Anspruch 6, wobei es sich bei dem bakteriostatischen Pharmakon, das eine Sulfanilamidogruppe enthält, um wenigstens eine Art handelt, die aus der Gruppe ausgewählt ist, die aus Sulfamethoxazol, Sulfamonomethoxin, Sulfisoxazol, Sulfisomidin und pharmakologisch annehmbaren Salzen davon besteht.

9. Verfahren gemäß Anspruch 6, wobei es sich bei dem bakteriostatischen Pharmakon, das eine Sulfanilamidogruppe enthält, um Sulfamethoxazol oder ein Natriumsalz davon handelt.

10. Verwendung eines bakteriostatischen Pharmakons, das eine Sulfanilamidogruppe enthält, zur Herstellung einer lichtstabilen wässrigen Lösung von Pranoprofen oder eines pharmakologisch annehmbaren Salzes davon.

## Revendications

1. Solution aqueuse contenant du pranoprofène ou un sel pharmacologiquement acceptable de celui-ci, et un agent pharmaceutique bactériostatique contenant un groupe sulfanilamido.

2. Solution aqueuse suivant la revendication 1, dans laquelle le pranoprofène ou un sel pharmacologiquement acceptable de celui-ci est contenu à une concentration de pranoprofène de 0,01-2,0 % en poids/volume, et l'agent pharmaceutique bactériostatique contenant un groupe sulfanilamido à une concentration de 2,0-4,0 % en poids/volume.

3. Solution aqueuse suivant la revendication 1, dans laquelle l'agent pharmaceutique bactériostatique contenant un groupe sulfanilamido est constitué d'au moins un type choisi dans le groupe comprenant le sulfaméthoxazole, la sulfamonométhoxine, le sulfisoazole, la sulfisomidine et leurs sels pharmacologiquement acceptables.

4. Solution aqueuse suivant la revendication 1, dans laquelle l'agent pharmaceutique bactériostatique contenant un groupe sulfanilamido est le sulfaméthoxazole ou un sel sodique de celui-ci.

5. Solution aqueuse suivant la revendication 1, qui est une goutte oculaire.

6. Procédé de fabrication de pranoprofène ou d'un sel pharmacologiquement acceptable de celui-ci stable à la lumière dans une solution aqueuse, qui comprend l'addition d'un agent pharmaceutique bactériostatique contenant un groupe sulfanilamido à la solution aqueuse contenant du pranoprofène ou un sel pharmacologiquement acceptable de celui-ci.

7. Procédé suivant la revendication 6, dans lequel l'agent pharmaceutique bactériostatique contenant un groupe sulfanilamido est ajouté à une concentration de 2,0-4,0 % en poids/volume à la solution aqueuse comprenant du pranoprofène ou un sel pharmacologiquement acceptable de celui-ci à une concentration de pranoprofène de 0,01-2,0 % en poids/volume.

8. Procédé suivant la revendication 6, dans lequel l'agent pharmaceutique bactériostatique contenant un groupe sulfanilamido est constitué d'au moins un type choisi dans le groupe comprenant le sulfaméthoxazole, la sulfamonométhoxine, le sulfisoxazole, la sulfisomidine et leurs sels pharmacologiquement acceptables.

9. Procédé suivant la revendication 6, dans lequel l'agent pharmaceutique bactériostatique contenant un groupe sulfanilamido est le sulfaméthoxazole ou un sel sodique de celui-ci.

10. Utilisation d'un agent pharmaceutique bactériostatique contenant un groupe sulfanilamido pour la fabrication de pranoprofène ou d'un sel pharmacologiquement acceptable de celui-ci stable à la lumière dans une solution aqueuse.
